# EUROPÄISCHE PATENTSCHRIFT

(11) **EP 1 294 284 B1**
(45) Veröffentlichungstag und Bekanntmachung des Hinweises auf die Patenterteilung: **01.12.2004**
(21) Anmeldenummer: 01951429.8
(22) Anmeldetag: 26.06.2001
(51) Int. Cl.: A61B 6/00

(54) **MODULARES RÖNTGENDIAGNOSTIKGERÄT**
MODULAR X-RAY DIAGNOSTIC APPLIANCE
APPAREIL DE DIAGNOSTIC MODULAIRE A RAYONS X

(30) Priorität: 29.06.2000 DE 10031736
(43) Veröffentlichungstag der Anmeldung: 26.03.2003
(73) Patentinhaber: SIEMENS AKTIENGESELLSCHAFT, 80333 München (DE)
(72) Erfinder: FADLER, Franz, 91077 Hetzles (DE); KAUL, Karlheinz, 91077 Neunkirchen (DE); LEIDENBERGER, Stefan, 91090 Effeltrich (DE); LINK, Heinz-Joachim, 91052 Erlangen (DE); LIEGL, Hans, 91058 Erlangen (DE); BRENDEL, Frank, 01640 Coswig (DE); PIEGER, Konrad, 91356 Kirchehrenbach (DE)
(86) Internationale Anmeldenummer: PCT/DE2001/002338
(87) Internationale Veröffentlichungsnummer: WO 2002/000116

(56) Entgegenhaltungen:
- EP-A- 0 251 487
- FR-A- 2 616 650

## Beschreibung

Es sind unterschiedliche Röntgendiagnostikgeräte bekannt, die, je nachdem ob sich der Röntgenstrahler ober- oder unterhalb einer Lagerungsplatte für ein Untersuchungsobjekt befindet, als Ober- oder Untertisch-Röntgendiagnostikgerät gekennzeichnet sind. Dem Röntgenstrahler kann ein Strahlenempfänger zugeordnet sein, der als Röntgenfilm, als Bildverstärker oder als Festkörperdetektor ausgebildet ist. Es ist bekannt, den Röntgenstrahler in Bezug zum Strahlenempfänger oder den Strahlenempfänger in Bezug zum Röntgenstrahler oder sowohl den Röntgenstrahler als auch den Strahlenempfänger verstellbar zu lagern, so dass ein Untersuchungsobjekt beispielsweise aus unterschiedlichen Richtungen durchstrahlt werden kann. Hierzu ist es beispielsweise bekannt, den Röntgenstrahler und den Strahlenempfänger einander gegenüberliegend an den Enden eines C-Bogens zu lagern. Bei diesen Röntgendiagnostikgeräten kann die Lagerungsplatte ortsfest oder beispielsweise an einem Sockel verstellbar gelagert sein, so dass sie in der Höhe und/oder um eine Schwenkachse und/oder entlang ihrer Längs- und/oder Querachse verstellbar ist. Ferner ist es bekannt, einen Röntgenstrahler, den Strahlenempfänger und die Lagerungsplatte an einer Boden- oder Deckensäule jeweils als separates Teil zu lagern. Die als Unter- oder Obertisch ausgeführten Röntgendiagnostikgeräte bilden jeweils eine Einheit und werden für sich aus individuellen Teilen zusammengesetzt.

EP 0 251 487 A offenbart ein modulares Röntgendiagnostikgerät, wobei verschiedene Komponente an verschiedenen Trägern gekoppelt werden.

Aufgabe der Erfindung ist es, unterschiedliche Röntgendiagnostikgeräte unter Verwendung einheitlicher oder gemeinsamer Teile herstellen zu können, um somit den Kostenaufwand zu reduzieren. Hierzu soll auch ein Verfahren angegeben werden.

Die erstgenannte Aufgabe wird erfindungsgemäß durch den Gegenstand des Patentanspruches 1 gelöst.

Vorteil der Erfindung ist, dass das Röntgendiagnostikgerät als modulares System ausgeführt ist, das einen Grundträger als erstes Modul, zumindest einen Träger als zweites Modul für den Grundträger und zumindest eine Komponente einer Aufnahmeeinrichtung als drittes Modul aufweist, wobei am Grundträger Trägerkoppelmittel zum Ankoppeln des Trägers ausgebildet sind und wobei am Grundträger Komponentenkoppelmittel zum Ankoppeln der Komponente ausgebildet sind. Das modulare Röntgendiagnostikgerät weist zudem eine Lagerungsvorrichtung für ein Untersuchungsobjekt als viertes Modul auf. Am Grundträger sind Lagerungsvorrichtungskoppelmittel zum Ankoppeln der Lagerungsvorrichtung ausgebildet. Bei dem modularen Röntgendiagnostikgerät nach der Erfindung ist auch ein Längswagen als fünftes Modul vorgesehen, das oberhalb des Grundträgers angeordnet ist und das mit einem Fest- und/ oder Loslager in Verbindung steht.

Dieser modulare Aufbau ermöglicht es, unterschiedliche Träger oder Komponenten an dem selben Grundträger anzuordnen, so dass die Ausbildung unterschiedlicher Röntgendiagnostikgeräte möglich ist.

Die Komponentenkoppelmittel umfassen ein am Grundträger angeordnetes Fest- und/oder ein Loslager, so dass die Komponenten zu deren Verstellung durch das Festlager eine exakte Führung und durch das Loslager eine weitere Abstützung erhalten. Zudem ist eine solche Ausgestaltung kostengünstig.

Die Anbringung des Längswagens auf der Oberseite des Grundträgers hat den Vorteil, dass die Seitenflächen des Grundträgers zur Ankopplung vieler verschiedener weiterer Module verwendbar sind. Dabei ist es vorteilhaft, falls die Führungsschiene des Loslagers geodätisch neben der des Festlagers angeordnet ist.

Es ist vorteilhaft, wenn die Lagerungsvorrichtungskoppelmittel an zumindest einer Stirnseite des Grundträgers ausgebildet sind. Es kann ein Röntgendiagnostikgerät in Verbindung mit dem selben Grundträger gebildet werden, das auch eine Lagerungsvorrichtung für ein Untersuchungsobjekt aufweist.

Der Grundträger ist beispielsweise als Holm ausgebildet. Er hat im Querschnitt insbesondere die Form eines - vorzugsweise flachen und/oder liegenden - Rechtecks.

Soll die Komponente verstellbar sein, so ist es vorteilhaft, wenn am Grundträger eine Antriebseinrichtung für die Komponente, insbesondere eine Antriebseinrichtung auch für unterschiedliche Komponenten, vorgesehen ist, die das Personal bei der Verstellung der Komponente zumindest unterstützt und vorzugsweise die Verstellung steuerbar ist. Dabei kann die Antriebseinrichtung mit dem Längswagen in Verbindung stehen.

Für den modulhaften Ausbau des Röntgendiagnostikgeräts ist es besonders vorteilhaft, falls ein Motor der Antriebseinrichtung - und ggf. ein Motor einer weiteren Antriebseinrichtung - innerhalb des Grundträgers angeordnet ist. Dadurch bleiben an der Außenseite viele freie Flächen, die als Koppelstellen für potentielle Module dienen können.

Vorzugsweise ist am Grundträger ein weiteres Festlager angeordnet, an dem weitere Module angreifen. Vorzugsweise ist an dem weiteren Festlager ein Strahlenempfänger angeordnet und/oder die Lagerungsvorrichtung für das Untersuchungsobjekt vorzugsweise verstellbar gelagert. Zur weiteren Abstützung des Strahlenempfängers und/oder der Lagerungsvorrichtung ist ein weiteres Loslager vorgesehen, das an einem zur Längsachse des Grundträgers zumindest annähernd parallel ausgerichteten und zum Grundträger beabstandeten Längsholm angeordnet ist. Durch die weitere Abstützung am Loslager ist eine präzise Führung gegeben. Zudem ist die Ausgestaltung des Festlagers hinsichtlich dessen Konstruktion weniger aufwendig. Vorzugsweise steht der Längsholm über einen Querholm mit dem Grundträger in Verbindung, insbesondere über an den an der Stirnseite des Grundträgers ausgebildeten Lagerungskoppelmitteln. Es wird somit ein vom Grundträger ausgehender Rahmen für den Strahlenempfänger und/oder die Lagerungsvorrichtung gebildet, deren Stabilität dadurch erhöht werden kann, dass ein weiterer Querholm an den an der weiteren Stirnseite des Grundträgers ausgebildeten Lagerungskoppelmitteln angreift. Damit der Strahlenempfänger und/oder die Lagerungsvorrichtung zumindest auch unterstützt verstellbar sind, ist es vorteilhaft, wenn eine weitere Antriebseinrichtung hierfür am Grundträger angeordnet ist.

Oberhalb des Längswagens kann über einen Kompressionsturm ein Strahlenempfänger und unterhalb des Längswagens zum Grundträger und zum Längswagen beabstandet ein Strahlenquelle vorgesehen sein. Es wird somit ein Untertisch-Röntgendiagnostikgerät aus Modulen gebildet.

Ist oberhalb des Längswagens über eine am Längswagen angeordnete galgenförmige Säule eine Strahlenquelle angeordnet, so wird in modularer Weise ein Obertisch-Röntgendiagnostikgerät gebildet.

Ein C-Bogen-Röntgendiagnostikgerät kann in modularer Weise dadurch gebildet werden, dass am Längswagen eine Halterung für einen C-Bogen gelagert ist.

Als Träger für insbesondere den Grundträger eignet sich ein Deckenstativ oder ein Sockel, der sich beispielsweise am Boden des Untersuchungsraumes abstützt.

Über den Sockel können vorteilhaft eine Höhenverstellung des Grundträgers und eine Verschwenkung um eine horizontale Achse bewirkt werden. In vorteilhafter Weise sind an der Unterseite und/oder wenigstens einer der Seitenwände des Grundträgers Trägerkoppelmittel vorgesehen, über die der entweder unterhalb des Grundträgers angeordnete Sockel oder seitlich neben dem Grundträger angeordnete Sockel über ein Verbindungselement an den Trägerkoppelmitteln angreift. Es kann somit ein Röntgendiagnostikgerät in Verbindung mit unterschiedlichen Tragmitteln, die jeweils auch als Module ausgebildet sind, hergestellt werden.

Weitere vorteilhafte Ausgestaltungen sind in den Unteransprüchen wiedergegeben.

Das erfindungsgemäße Röntgendiagnostikgerät zeichnet sich insbesondere also durch modulare Bauweise aus, die es gestattet, dass mit denselben Modulen oder Komponenten unterschiedliche Röntgendiagnostikgeräte hergestellt werden können. Es kann somit auf Basiskomponenten zurückgegriffen werden, wodurch sich insbesondere die Herstellungskosten reduzieren lassen.

Die zweitgenannte Aufgabe wird gelöst durch die Merkmale des Anspruchs 29. Vorteilhafte Ausführungen des Verfahrens sind in den diesbezüglichen Unteransprüchen angegeben.

Weitere Vorteile und Einzelheiten der Erfindung ergeben sich aus der nachfolgenden Beschreibung eines Ausführungsbeispieles anhand der Zeichnungen in Verbindung mit den Unteransprüchen. Es zeigen:
- Fig. 1: die Schnittstelle zwischen dem Grundträger und einem Ausleger;
- Fig. 2: die Schnittstelle zwischen dem Grundträger und einem Kippfuß;
- Fig. 3: die in Fig. 2 dargestellte Schnittstelle in einer Seitenansicht;
- Fig. 4: die Schnittstelle zwischen dem Grundträger und einem Obertisch-Zielgerät;
- Fig. 5: die Schnittstelle zwischen dem Grundträger und einer Obertisch-Strahlersäule;
- Fig. 6: die Schnittstelle zwischen dem Grundträger und einem Längswagen eines Untertisch-Systems;
- Fig. 7: die Schnittstelle zwischen dem Grundträger und einem C-Bogen-System;
- Fig. 8: die Schnittstelle zwischen dem Grundträger und einer als Tischplatte ausgebildeten Lagerungsvorrichtung;
- Fig. 9: eine Gesamtansicht eines erfindungsgemäßen modularen Röntgendiagnostikgeräts mit einem Kippfuß.

Röntgendiagnostikgeräte bestehen aus mehreren, unterschiedlichen Komponenten, die in Abhängigkeit von dem beabsichtigten Einsatzzweck miteinander kombiniert werden. Der konkrete Aufbau des Geräts wird vom Hersteller abhängig von der vom Abnehmer gegebenen Gerätespezifikation bzw. dem geforderten Leistungsprofil konzeptioniert und die relevanten Teile zusammengestellt. In einer Grundversion des Röntgendiagnostikgeräts sind alle unbedingt notwendigen Komponenten vorhanden. Bei höherwertigen Ausstattungen sind zusätzlich zu der Grundversion weitere Optionen eingebaut. Bei einem "High-End-Gerät" sind sämtliche Optionen verwirklicht.

Röntgendiagnostikgeräte unterscheiden sich auch in ihrem Aufbau, wobei grundsätzlich zwischen Obertisch- und Untertischausführungen unterschieden wird, je nachdem, an welcher Stelle der Strahler angebracht ist.

Um alle möglichen Kombinationen und Aufbauten machbar aufbauen zu können, wird bei dem erfindungsgemäßen Röntgendiagnostikgerät ein Plattformkonzept verwirklicht, das sowohl für Untertisch- als auch für Obertischgeräte verwendet werden kann. Die zentrale Komponente jedes Systems ist ein weitgehend standardisierter Grundträger mit mehreren Schnittstellen zum Anbau weiterer Komponenten. Der Grundträger wird generell allen aufbaubaren Röntgendiagnostikgeräten zugrunde gelegt, je nach gewünschter Ausführung werden beliebige Komponenten angebaut. Ein standardisierter Träger liegt also dem ganzen modularen System zugrunde. Die Schnittstellen können in der einfachsten Form als Gewindebohrungen ausgeführt sein, an die weitere Koppelmittel anschraubbar sind. In einer Basisausstattung umfasst das Röntgendiagnostikgerät neben dem Grundträger einen kippbaren Tisch mit einer motorisch angetriebenen Tischplatte sowie ein Zielgerät. Durch das Plattformkonzept lässt sich die Teileanzahl bis zu 70 % verringern, dennoch können alle Varianten modular zusammengebaut werden.

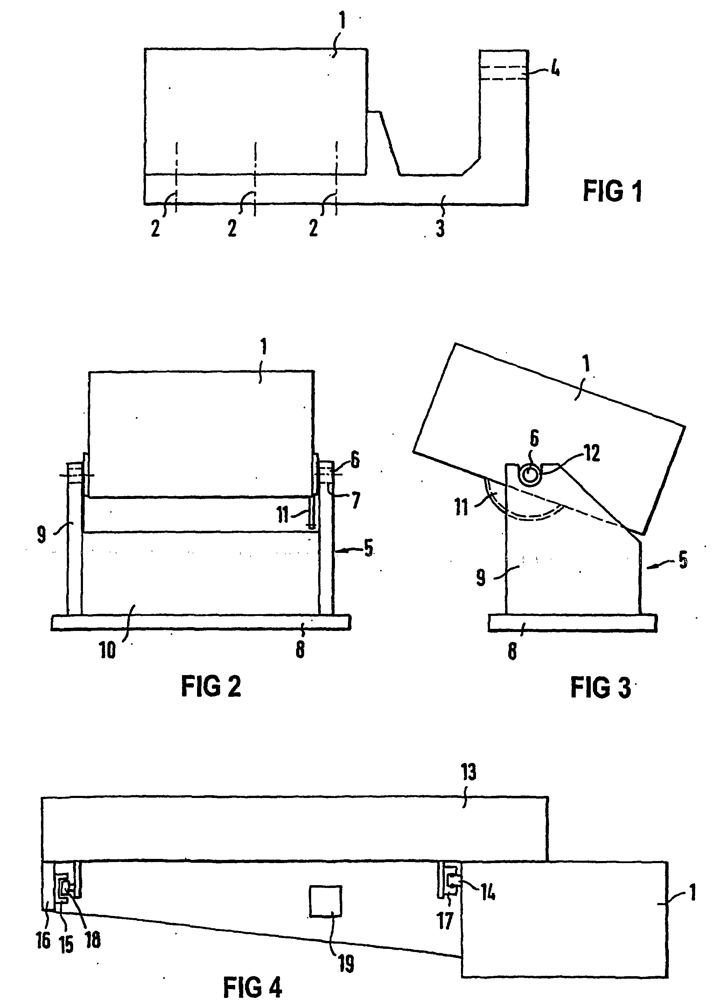

In den Figuren 1-8 werden die Schnittstellen zwischen dem Grundträger und Anbaukomponenten beschrieben.

Fig. 1 zeigt den Grundträger 1 des erfindungsgemäßen modularen Röntgendiagnostikgeräts, der als Modul aufgebaut ist und die zentrale Komponente des Geräts darstellt. Der Grundträger 1 ist bei jedem modularen Röntgendiagnostikgerät zwingend vorhanden, sämtliche weiteren Komponenten sind an dem Grundträger 1 unmittelbar oder mittelbar angeordnet. Bei dem in Fig. 1 dargestellten Ausführungsbeispiel sind an der Unterseite des Grundträgers 1 sowie auf einer Seite Trägerkoppelmittel angeordnet, die als Schraubverbindungen 2 ausgebildet sind. Über diese Schraubverbindungen 2 wird der Grundträger 1 mit einem Ausleger 3 verschraubt. Der Ausleger 3 besteht im Wesentlichen aus einem waagerechten, unterhalb des Grundträgers 1 angeordneten Abschnitt, der seitlich hervorragt und einem senkrechten Abschnitt, der in seinem oberen Bereich eine Lageraufnahme 4 aufweist. Über die Lageraufnahme 4 kann der Ausleger 3 an einem Sockel drehbar gelagert werden. Zusätzlich kann eine Höhenverstellung des Auslegers 3 vorgesehen sein, so dass sich ein Hub-Kipp-Fuß ergibt. Es kommen drei verschiedene Fußvarianten zum Einsatz, die einen Schwenkbereich von -20° bis +90°, -45° bis 90° und +/- 90° ermöglichen. Dabei wird der Grundträger 1 gemeinsam mit dem Ausleger 3 um eine horizontale, durch die Lageraufnahme 4 laufende Achse verschwenkt. Die Tatsache, dass die Lageraufnahme 4 des Auslegers 3 neben dem Grundträger 1 angeordnet ist, führt dazu, dass der Grundträger 1 von allen Seiten gut zugänglich ist.

Die in Fig. 2 dargestellte Schnittstelle zwischen dem Grundträger 1 und einem Kippfuß 5 stellt eine Alternative zu der in Fig. 1 dargestellten Schnittstelle dar. Die an dem Grundträger 1 ausgebildeten Trägerkoppelmittel sind Lagerbolzen 6, die einander gegenüberliegend an den Längsseiten des Grundträgers 1 angeordnet sind. Die Lagerbolzen 6 werden an den Grundträger 1 angeschraubt bzw. angeschweißt. Sie dienen zur Lagerung des Grundträgers 1 in Lageraufnahmen 7 eines Kippfußes 5. Der Kippfuß 5 umfasst eine Bodenplatte 8 sowie zwei parallele, zueinander beabstandete Seitenwände 9 sowie die Lageraufnahmen 7 im oberen Bereich der Seitenwand 9. Zwischen den beiden Seitenwänden 9 des Kippfußes 5 befindet sich unter einer Abdeckung 10 ein nicht dargestellter Antrieb, der mit einem an dem Grundträger 1 angeordneten und starr damit verbundenen Zahnsegment 11 zusammenwirkt. Durch den Antrieb des Zahnsegments 11 lässt sich eine Drehung des Grundträgers 1 um die waagerechte, durch die Lageraufnahme 7 verlaufende Achse bewirken.

Die Anordnung von Fig. 2 ist in Fig. 3 in einer Seitenansicht dargestellt. In der Lageraufnahme 7 ist ein Kugellager 12 angeordnet, das zur Aufnahme des Lagerbolzens 6 dient. Durch den Kippfuß 5 lassen sich der Grundträger 1 und daran angeordnete weitere Bauteile in einem Schwenkbereich von wenigstens +90° bis -20° kippen. An Stelle des hier gezeigten Zahnsegments 11 kann auch ein Zahnrad, ein Zahnriemen oder ein Getriebe zur Verstellung benutzt werden. In einer Weiterbildung der Erfindung kann in den Kippfuß 5 zusätzlich eine Hubeinheit integriert werden, um eine Höhenverstellung des Grundträgers 1 zu ermöglichen. Die Schnittstelle zum Grundträger ist dieselbe, es kann aber auch eine zusätzliche Schnittstelle vorgesehen sein. Das Auf- und Absteigen wird dem Patienten auf diese Weise wesentlich erleichtert, ebenso wird seine Zugänglichkeit verbessert. Eine derartige Hubeinheit enthält eine Getriebeeinheit, die z. B. über einen Kettenantrieb vertikal verstellbar ist. Andere Antriebe sind auch denkbar.

Fig. 4 stellt eine Schnittstelle zwischen dem Grundträger 1 und einem Obertisch-Zielgerät 13 dar. Dazu sind an dem Grundträger 1 seitlich Komponentenkoppelmittel zum Ankoppeln der Komponente ausgebildet, die in dem dargestellten Ausführungsbeispiel als Kombination aus einer Festlagerführung 14 und einer Loslagerführung 15 ausgebildet sind. Die Festlagerführung 14 ist an dem Grundträger 1 seitlich angeschraubt. Die Loslagerführung 15 ist an einem Holm 16 befestigt. Die Anordnung des Fest- und des Loslagers kann auch umgekehrt gewählt sein. Der Holm 16 ist als Längsholm ausgebildet und erstreckt sich parallel zu der Längsachse des Grundträgers 1. Die Verbindung zwischen dem Holm 16 und dem Grundträger 1 erfolgt über in Fig. 4 nicht gezeigte Querholme, die beidseitig an den Enden des Holms 16 angeordnet sind und zusammen mit dem Holm 16 einen Träger bilden. Das Festlager 14 wirkt mit entsprechenden Lagerkomponenten 17 zusammen, die an der Unterseite des Obertisch-Zielgeräts 13 angeordnet sind. Bei dem Festlager kann es sich beispielsweise um einen Kugelumlaufwagen oder einen Gleitführungswagen handeln. Analog wirkt die Loslagerführung 15 mit Lagerkomponenten 18 zusammen, die beispielsweise als Rollenlager ausgebildet sein können. Mit dieser Lagerung wird eine exakte Führung durch das Festlager und eine weitere Abstützung durch das Loslager erzielt. Zwischen den Führungen verläuft ein Antriebsstrang mit einem Antrieb 19, der bevorzugt als Kette, aber auch als Spindel, Zahnriemen oder Zahnstange ausgebildet sein kann. Über den Antrieb 19 lässt sich die Längsverschiebung des Obertisch-Zielgeräts 13 motorisch durchführen. Alternativ zu dem Obertisch-Zielgerät 13 kann auch eine Untertisch-Kassettenlade vorgesehen sein. Die Lagerführungen 14, 15 ermöglichen die Befestigung verschieden großer Holme 16 und Träger.

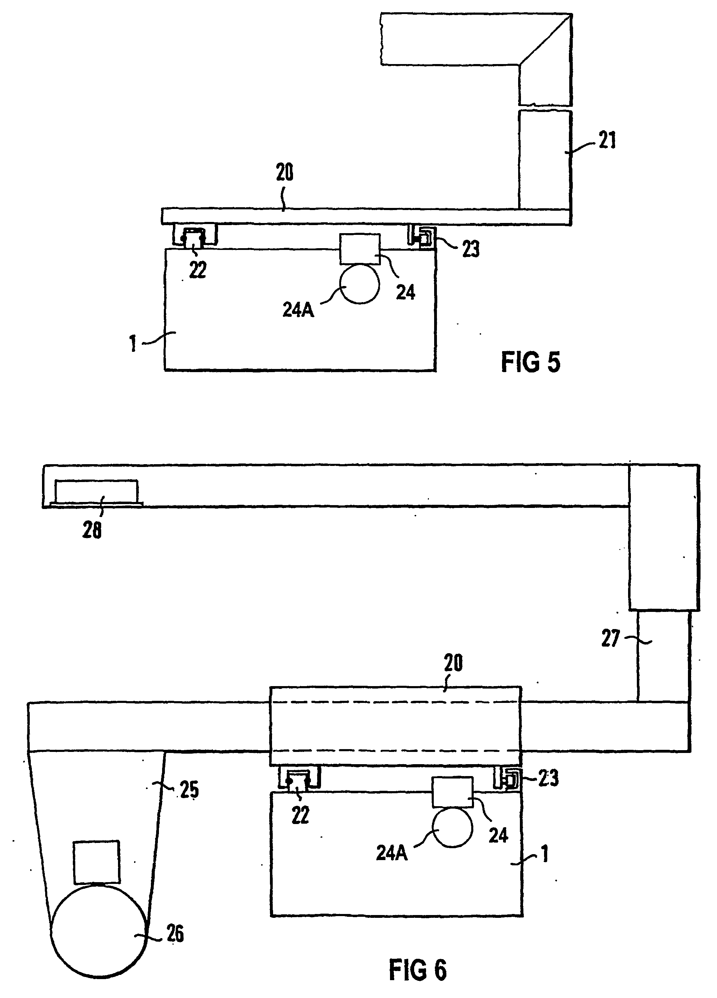

Eine weitere an dem Grundträger 1 angeordnete Schnittstelle ist in Fig. 5 dargestellt. Es handelt sich dabei um eine Schnittstelle zu einem Längswagen 20 mit einer daran angeordneten Strahlersäule 21. Am oberen Ende der Strahlersäule 21 ist ein in Fig. 5 nicht dargestellter Strahlenquelle angeordnet. Zur Lagerung an dem Grundträger 1 weist dieser Komponentenkoppelmittel auf, die als Festlagerführung 22 und Loslagerführung 23 ausgebildet sind. Die Lagerführungen entsprechen den in Fig. 4 dargestellten Lagerführungen 14 und 15. Das Festlager 22 besteht z.B. aus einem Kugelumlaufwagen oder einem Gleitführungswagen, der mit einer entsprechenden Schiene zusammenwirkt. Bei dem Loslager 23 handelt es sich um ein Rollenlager, das beispielsweise in einem dazu passenden C-Profil läuft. Die Lagerführungen 22, 23 werden an dem Grundträger 1 durch Schraubverbindungen angebracht, so dass diese bei Bedarf einfach ausgetauscht werden können. Innerhalb des Grundträgers 1 befindet sich ein Motor 24A eines nur schematisch gezeichneten Antriebs 24, über den die Verschiebung des Längswagens 20 und damit der Strahlersäule 21 in Längsrichtung des Grundträgers 1 erfolgt. Der Träger und der Längswagen 20 lassen sich unabhängig voneinander verschieben.

Eine Modifikation des Ausführungsbeispiels von Fig. 5 zeigt Fig. 6. Der Grundträger 1 mit den Lagerungen 22, 23 und dem Längswagen 20 entspricht dabei identisch dem vorherigen Ausführungsbeispiel. Der Längswagen 20 weist auf einer Seite eine Verlängerung auf, an die sich ein unterhalb des Längswagens angeordneter Ausleger 25 anschließt, an dem ein Strahlenquelle 26 befestigt ist. Der Strahlenquelle 26 befindet sich neben dem Grundträger 1 unterhalb des Längswagens 20. An der gegenüberliegenden Seite des Längswagens 20 ist ein vertikaler Kompressionsturm 27 angeordnet, der an seinem oberen Ende ein Zielgerät und/oder einen Bildempfänger 28 trägt. Der Strahlenquelle 26 ist auf den Bildempfänger 28 ausgerichtet. Das Zielgerät kann auch einen Röntgenbildverstärker oder einen Festkörperdetektor enthalten. Insgesamt stellt diese Anordnung ein modulares Untertisch-Röntgendiagnostikgerät dar, das über den Längswagen 20 in Längsrichtung relativ zum Grundträger 1 verfahrbar ist.

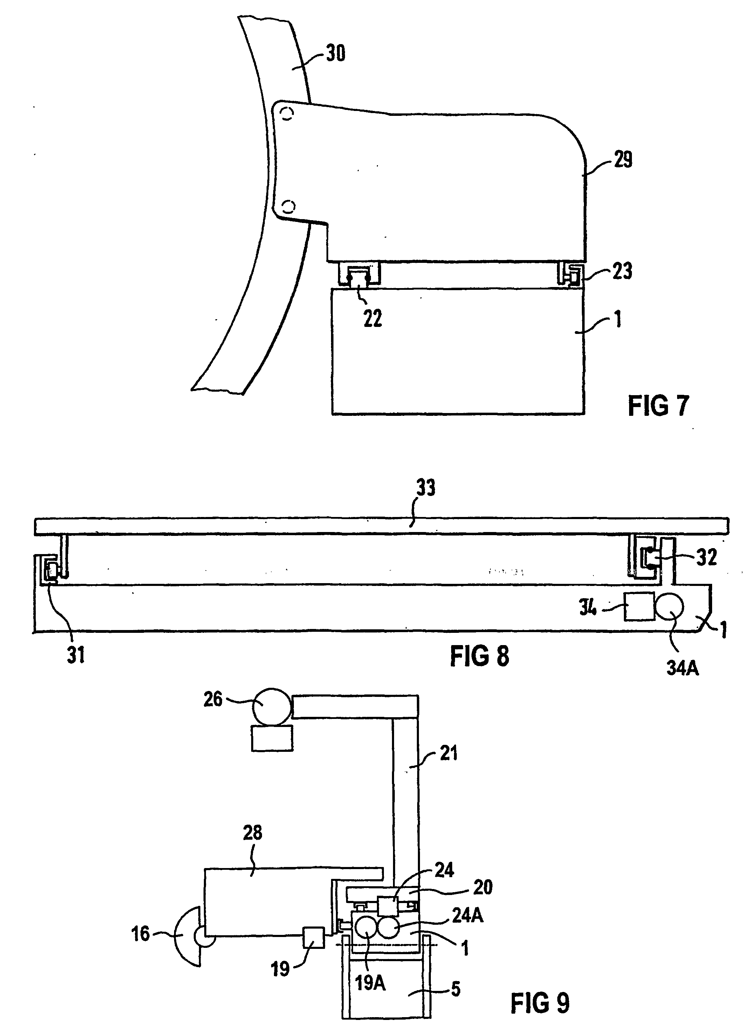

Eine weitere Erfindungsalternative ist in Fig. 7 dargestellt. Wie in dem Ausführungsbeispiel von Fig. 6 weist der Grundträger 1 an seiner Oberseite Lagerführungen 22, 23 auf, die über eine einen Längswagen darstellende Halterung 29 zur Lagerung und Führung eines C-Bogen-Systems 30 vorgesehen sind. Die Halterung 29, die daneben auch eine Querverschiebung und/oder Drehbewegung des an ihr gehalterten C-Bogen zulassen kann, ist fest mit dem C-Bogen 30 verbunden, beispielsweise verschraubt und ermöglicht dadurch eine Längsverschiebung des C-Bogen-Systems. Die Anordnung der Führungen und des Antriebs entspricht der des vorherigen Ausführungsbeispiels.

Alternativ kann es auch vorgesehen sein, die Halterung 29 an einem gesonderten Längswagen 20 zu befestigen, so dass die Komponenten des Fest- und Loslagers 22, 23 zwischen dem Grundträger 1 und dem Längswagen 20 angeordnet sind.

Fig. 8 zeigt eine Längsseite des Grundträgers 1 in einer Seitenansicht. An beiden Stirnseiten des Grundträgers 1 sind Lagerungsvorrichtungskoppelmittel angeordnet, die als Loslagerführung 31 und Festlagerführung 32 ausgebildet sind. Die Führungen 31, 32 dienen zur Führung einer Lagerungsvorrichtung für ein Untersuchungsobjekt, die als Tischplatte 33 ausgebildet ist. Über einen in dem Grundträger 1 angeordneten Antrieb 34 (mit Motor 34A) kann die Tischplatte 33 motorisch bewegt werden. Zusammen mit der Lagerung 22, 23 und dem Anrieb 24 kann die Tischplatte 33 sowohl in Längs- als auch in Querrichtung verschoben werden.

Fig. 9 zeigt ein besonders geeignetes Ausführungsbeispiel der Erfindung in einer Gesamtansicht, bei dem die in den Fig. 2, 4 und 5 beschriebenen Schnittstellen zwischen dem Grundträger 1 und weiteren Modulen verwirklicht sind. Unterhalb des Trägers 1 befindet sich der Kippfuß 5, der eine Verschwenkung des Röntgendiagnostikgeräts ermöglicht. Oberhalb des Grundträgers 1 ist die Strahlersäule 21 mit dem Strahlenquelle 26 angeordnet. Seitlich in Bezug auf den Grundträger 1 ist das Zielgerät/Bildempfänger 28 in Längsrichtung verschieblich gelagert. Eine zusätzliche Abstützung ergibt sich durch den Holm 16. Durch den mit dem Bildempfänger 28 zusammenwirkenden Antrieb 19 (mit Motor 19A im Grundträger 1) und den mit der Strahlersäule 21 zusammenwirkenden Antrieb 24 (mit Motor 24A im Grundträger 1) können beide Module unabhängig voneinander bewegt werden. Insgesamt ergibt sich auf diese Weise ein modular aufgebautes Röntgendiagnostikgerät, das aus unterschiedlichen Komponenten besteht, die in der Art eines Baukastensystems zusammengestellt sind.

## Patentansprüche

1. Modulares Röntgendiagnostikgerät, aufweisend folgende Module:
a) als erstes Modul einen Grundträger (1),
b) als zweites Modul zumindest einen Träger für den Grundträger (1), wobei am Grundträger (1) Trägerkoppelmittel (6) zum Ankoppeln des Trägers ausgebildet sind,
c) als drittes Modul zumindest eine Komponente einer Röntgenbild-Aufnahmeeinrichtung, wobei am Grundträger (1) Komponentenkoppelmittel zum Ankoppeln der Komponente ausgebildet sind,
d) als viertes Modul eine Lagerungsvorrichtung (33) für ein Untersuchungsobjekt, wobei am Grundträger (1) Lagerungsvorrichtungskoppelmittel (31,32) zum Ankoppeln der Lagerungsvorrichtung (33) ausgebildet sind, und
e) als fünftes Modul einen Längswagen (20), der oberhalb des Grundträgers (1) angeordnet ist und an dem die Komponente angebracht ist, wobei die Komponentenkoppelmittel ein am Grundträger (1) angeordnetes Fest- und/oder ein Loslager (22, 23) umfassen, die mit dem Längswagen (20) in Verbindung stehen.

2. Modulares Röntgendiagnostikgerät nach Anspruch 1,
wobei die Lagerungsvorrichtungskoppelmittel (31,32) an zumindest einer Stirnseite des Grundträgers (1) ausgebildet sind.

3. Modulares Röntgendiagnostikgerät nach einem der Ansprüche 1 bis 2,
wobei unterschiedliche Komponenten an denselben Komponentenkoppelmitteln am Grundträger (1) ankoppelbar sind.

4. Modulares Röntgendiagnostikgerät nach einem der Ansprüche 1 bis 3,
wobei die Komponente ein Strahlenempfänger (28) ist.

5. Modulares Röntgendiagnostikgerät nach einem der Ansprüche 1 bis 4,
wobei am Grundträger (1) eine Antriebseinrichtung (24) angeordnet ist, die mit dem Längswagen (20) in Verbindung steht.

6. Modulares Röntgendiagnostikgerät nach Anspruch 5,
wobei ein Motor (24A) der Antriebseinrichtung (24) innerhalb des Grundträgers (1) angeordnet ist.

7. Modulares Röntgendiagnostikgerät nach einem der Ansprüche 1 bis 6,
wobei die Komponentenkoppelmittel ein am Grundträger (1) angeordnetes weiteres Fest- und ein weiteres Loslager (14,15) umfassen.

8. Modulares Röntgendiagnostikgerät nach einem der Ansprüche 1 bis 7,
wobei am Grundträger (1) eine weitere Antriebseinrichtung (19) für die Komponente angeordnet ist.

9. Modulares Röntgendiagnostikgerät nach Anspruch 8,
wobei über die weitere Antriebseinrichtung (19) unterschiedliche Komponenten verstellbar sind.

10. Modulares Röntgendiagnostikgerät nach einem der Ansprüche 7 bis 9,
wobei die weitere Antriebseinrichtung (19) zwischen dem Fest- und dem Loslager (14,15) angeordnet ist.

11. Modulares Röntgendiagnostikgerät nach Anspruch 9 oder 10,
wobei an einem anderen Festlager (32) die Lagerungsvorrichtung (33) für das Untersuchungsobjekt gelagert ist.

12. Modulares Röntgendiagnostikgerät nach Anspruch 11,
wobei die Lagerungsvorrichtung (33) am anderen Festlager (32) verstellbar ist.

13. Modulares Röntgendiagnostikgerät nach einem der Ansprüche 1 bis 12,
wobei die Lagerungsvorrichtung (33) oberhalb des Strahlenempfängers (28) angeordnet ist.

14. Modulares Röntgendiagnostikgerät nach einem der Ansprüche 1 bis 13,
wobei der Strahlenempfänger (13) und/oder die Lagerungsvorrichtung (33) an einem Loslager (15) angreifen, das an einem zur Längsachse des Grundträgers (1) zumindest annähernd parallel ausgerichteten und zum Grundträger (1) beabstandeten Längsholm (16) angeordnet ist.

15. Modulares Röntgendiagnostikgerät nach Anspruch 14,
wobei der Längsholm (16) über einen Querholm mit dem Grundträger (1) in Verbindung steht.

16. Modulares Röntgendiagnostikgerät nach Anspruch 15,
wobei der Querholm an den an der Stirnseite des Grundträgers (1) ausgebildeten Lagerungskoppelmitteln (31) angreift.

17. Modulares Röntgendiagnostikgerät nach Anspruch 15 oder 16,
wobei ein weiterer Querholm an den an der weiteren Stirnseite des Grundträgers ausgebildeten Lagerungskoppelmitteln (32) angreift und mit dem Längsholm (16) in Verbindung steht.

18. Modulares Röntgendiagnostikgerät nach einem der Ansprüche 1 bis 17,
wobei oberhalb des Längswagens (20) über einen am Längswagen (20) angeordneten Kompressionsturm (27) ein Strahlenempfänger (28) angeordnet ist, und
wobei ein unterhalb des Längswagens (20), zum Grundträger (1) beabstandet und am Längswagen (20) angeordneter Strahlenquelle (26) vorgesehen ist.

19. Modulares Röntgendiagnostikgerät nach einem der Ansprüche 1 bis 17,
wobei oberhalb des Längswagens (20) über eine am Längswagen (20) angeordnete galgenförmige Säule (21) ein Strahlenquelle (26) angeordnet ist.

20. Modulares Röntgendiagnostikgerät nach einem der Ansprüche 1 bis 19,
wobei am Längswagen (20) eine Halterung (29) für einen C-Bogen (30) gelagert ist, über die der C-Bogen (30) zumindest entlang seines Umfanges verstellbar ist.

21. Modulares Röntgendiagnostikgerät nach Anspruch 20,
wobei der C-Bogen (30) über die Halterung (29) um eine horizontale und/oder eine senkrechte Achse verstellbar ist.

22. Modulares Röntgendiagnostikgerät nach einem der Ansprüche 1 bis 21,
wobei der Träger als Deckenstativ ausgeführt ist.

23. Modulares Röntgendiagnostikgerät nach einem der Ansprüche 1 bis 21,
wobei der Träger als Sockel (5) ausgebildet ist.

24. Modulares Röntgendiagnostikgerät nach Anspruch 23,
wobei über den Sockel (5) eine Höhenverstellung des Grundträgers (1) bewirkbar ist.

25. Modulares Röntgendiagnostikgerät nach Anspruch 23 oder 24,
wobei über den Sockel (5) eine Verschwenkung des Grundträgers (1) um eine horizontale Achse bewirkbar ist.

26. Modulares Röntgendiagnostikgerät nach einem der Ansprüche 23 bis 25,
wobei der Sockel (5) an den an der Unterseite und/oder wenigstens einer der Seitenwände des Grundträgers (1) vorgesehenen Trägerkoppelmitteln (6) angreift.

27. Modulares Röntgendiagnostikgerät nach einem der Ansprüche 23 bis 26,
wobei der Sockel (5) unterhalb des Grundträgers (1) angeordnet ist.

28. Modulares Röntgendiagnostikgerät nach einem der Ansprüche 23 bis 26,
wobei der Sockel (5) seitlich neben dem Grundträger (1) angeordnet ist und über ein Verbindungselement (3) an den Trägerkoppelmitteln (2) angreift.

29. Herstellungsverfahren für unterschiedliche Serien von Röntgendiagnostikgeräten,
wobei für alle Serien ein baugleicher modulartiger Grundträger (1) und eine baugleiche modulartige Lagerungsvorrichtung (33) verwendet werden,
wobei jeder Grundträger (1) als zentrales Modul an unterschiedlichen Koppelstellen zur Ankopplung aller in den zu erstellenden Serien vorkommenden Module vorbereitet wird,
und wobei an dem Grundträger (1) die Lagerungsvorrichtung (33) und in Abhängigkeit von der jeweiligen Serie weitere Module angebracht werden,
wobei die weiteren Module einen Träger für den Grundträger (1) und/oder eine Komponente einer Röntgenbild-Aufnahmeeinrichtung und/oder einen Längswagen (20).

30. Herstellungsverfahren nach Anspruch 29,
wobei die Serien Serien voneinander unterschiedlicher Gerätetypen sind, insbesondere eine Obertisch-Röntgendiagnostikgeräteserie, eine Untertisch-Röntgendiagnostikgeräteserie und/oder eine C-Bogen-Röntgendiagnostikgeräteserie umfassen.

31. Herstellungsverfahren nach Anspruch 29 oder Anspruch 30,
wobei die Serien Serien des gleichen Gerätetyps mit unterschiedlich umfangreicher oder komfortabler Ausstattung sind.

## Claims

1. A modular X-ray diagnostic appliance, having the following modules:
a) as first module, a base support (1),
b) as second module, at least one support for the base support (1), support-coupling elements (6) being formed on the base support (1) for coupling the support thereto,
c) as third module, at least one component of an X-ray imaging device, component-coupling elements being formed on the base support (1) for coupling the component thereto,
d) as fourth module, a bearing device (33) for an object to be examined, bearing-device coupling elements (31, 32) being formed on the base support (1) for coupling the bearing device (33) thereto, and
e) as fifth module, a longitudinal carriage (20) which is arranged above the base support (1) and on which the component is mounted, the component-coupling elements comprising a fixed and/or floating bearing (22, 23) arranged on the base support (1) and connected to the longitudinal carriage (20).

2. The modular X-ray diagnostic appliance as claimed in Claim 1,
in which the bearing-device coupling elements (31, 32) are formed on at least one end face of the base support (1).

3. The modular X-ray diagnostic appliance as claimed in one of Claims 1 and 2,
in which different components can be coupled to the same component-coupling elements on the base support (1).

4. The modular X-ray diagnostic appliance as claimed in one of Claims 1 to 3,
in which the component is a radiation receiver (28).

5. The modular X-ray diagnostic appliance as claimed in one of Claims 1 to 4,
in which a drive device (24) is arranged on the base support (1) and is connected to the longitudinal carriage (20).

6. The modular X-ray diagnostic appliance as claimed in Claim 5,
in which a motor (24A) of the drive device (24) is arranged inside the base support (1).

7. The modular X-ray diagnostic appliance as claimed in one of Claims 1 to 6,
in which the component-coupling elements comprise a further fixed bearing (14) and a further floating bearing (15) arranged on the base support (1).

8. The modular X-ray diagnostic appliance as claimed in one of Claims 1 to 7,
in which a further drive device (19) for the component is arranged on the base support (1).

9. The modular X-ray diagnostic appliance as claimed in Claim 8,
in which different components can be adjusted via the further drive device (19).

10. The modular X-ray diagnostic appliance as claimed in one of Claims 7 to 9,
in which the further drive device (19) is arranged between the fixed bearing (14) and the floating bearing (15).

11. The modular X-ray diagnostic appliance as claimed in Claim 9 or 10,
in which the bearing device (33) for the object to be examined is mounted on another fixed bearing (32).

12. The modular X-ray diagnostic appliance as claimed in Claim 11,
in which the bearing device (33) can be adjusted on the other fixed bearing (32).

13. The modular X-ray diagnostic appliance as claimed in one of Claims 1 to 12,
in which the bearing device (33) is arranged above the radiation receiver (28).

14. The modular X-ray diagnostic appliance as claimed in one of Claims 1 to 13,
in which the radiation receiver (13) and/or the bearing device (33) engage on a floating bearing (15) which is arranged on a longitudinal bar (16) oriented at least approximately parallel to the longitudinal axis of the base support (1) and spaced apart from said base support (1).

15. The modular X-ray diagnostic appliance as claimed in Claim 14,
in which the longitudinal bar (16) is connected to the base support (1) via a transverse bar.

16. The modular X-ray diagnostic appliance as claimed in Claim 15,
in which the transverse bar engages on the bearing-coupling elements (31) formed on the end face of the base support (1).

17. The modular X-ray diagnostic appliance as claimed in Claim 15 or 16,
in which a further transverse bar engages on the bearing-coupling elements (32) formed on the other end face of the base support and is connected to the longitudinal bar (16).

18. The modular X-ray diagnostic appliance as claimed in one of Claims 1 to 17,
in which a radiation receiver (28) is arranged above the longitudinal carriage (20) via a compression tower (27) arranged on said longitudinal carriage (20), and
in which a radiation source (26) is provided below the longitudinal carriage (20) and is arranged on said longitudinal carriage (20) and spaced apart from the base support (1).

19. The modular X-ray diagnostic appliance as claimed in one of Claims 1 to 17,
in which a radiation source (26) is arranged above the longitudinal carriage (20) via a gallows-like column (21) arranged on said longitudinal carriage (20).

20. The modular X-ray diagnostic appliance as claimed in one of Claims 1 to 19,
in which a holder (29) for a C-bow (30) is mounted on the longitudinal carriage (20), via which the C-bow (30) is adjustable at least along its circumference.

21. The modular X-ray diagnostic appliance as claimed in Claim 20,
in which the C-bow (30) can be adjusted via the holder (29) about a horizontal and/or vertical axis.

22. The modular X-ray diagnostic appliance as claimed in one of Claims 1 to 21,
in which the support is designed as a ceiling mounting.

23. The modular X-ray diagnostic appliance as claimed in one of Claims 1 to 21,
in which the support is designed as a pedestal (5).

24. The modular X-ray diagnostic appliance as claimed in Claim 23,
in which the base support (1) can be adjusted in height via the pedestal (5).

25. The modular X-ray diagnostic appliance as claimed in Claim 23 or 24,
in which the base support (1) can be swivelled
about a horizontal axis via the pedestal (5).

26. The modular X-rav diagnostic appliance as claimed in one of Claims 23 to 25,
in which the pedestal (5) engages on the support-coupling elements (6) which are provided on the underside and/or at least one of the side walls of the base support (1).

27. The modular X-ray diagnostic appliance as claimed in one of Claims 23 to 26,
in which the pedestal (5) is arranged below the base support (1).

28. The modular X-ray diagnostic appliance as claimed in one of Claims 23 to 26,
in which the pedestal (5) is arranged laterally next to the base support (1) and engages on the support-coupling elements (2) via a connecting element (3).

29. A method for producing different series of X-ray diagnostic appliances,
in which a structurally identical modular base support (1) and a structurally identical modular bearing device (33) are used for all series,
in which each base support (1), as the central module, is prepared at different coupling points for coupling thereto of all the modules occurring in the series which are to be produced,
and in which the bearing device (33) and, depending on the particular series, further modules are arranged on the base support (1),
in which the further modules include a support for the base support (1) and/or a component of an X-ray imaging device and/or a longitudinal carriage (20).

30. The production method as claimed in Claim 29,
in which the series are series of mutually different appliance types, in particular a series of over-table X-ray diagnostic appliances, a series of under-table X-ray diagnostic appliances and/or a series of C-bow X-ray diagnostic appliances.

31. The production method as claimed in Claim 29 or Claim 30,
in which the series are series of the same appliance type which are comprehensively or comfortably equipped to differing degrees.

## Revendications

1. Appareil modulaire de diagnostic par rayons X, comprenant les modules suivants :
a) comme premier module, un support (1) de base,
b) comme deuxième module, au moins un support pour le support (1) de base, des moyens (6) de couplage de support étant formés sur le support (1) de base pour l'accouplement du support,
c) comme troisième module, au moins un composant d'un équipement enregistreur de radiographie, des moyens de couplage de composant étant formés sur le support (1) de base pour l'accouplement du composant,
d) comme quatrième module, un dispositif (33) de soutien pour un objet à examiner, des moyens (31, 32) de couplage de dispositif de soutien étant formés sur le support (1) de base pour l'accouplement du dispositif (33) de soutien,
e) comme cinquième module, un chariot (20) longitudinal qui est disposé au-dessus du support (1) de base et sur lequel le composant est installé, les moyens de couplage de composant comprenant un palier (22) fixe et/ou un palier (23) libre, qui sont disposés sur le support (1) de base et sont reliés au chariot (20) longitudinal.

2. Appareil modulaire de diagnostic par rayons X suivant la revendication 1, dans lequel les moyens (31, 32) de couplage de dispositif de soutien sont formés sur au moins un côté frontal du support (1) de base.

3. Appareil modulaire de diagnostic par rayons X suivant la revendication 1 ou 2, dans lequel des composants différents peuvent être couplés au support (1) de base par les mêmes moyens de couplage de composant.

4. Appareil modulaire de diagnostic par rayons X suivant l'une des revendications 1 à 3, dans lequel le composant est un récepteur (28) de rayons.

5. Appareil modulaire de diagnostic par rayons X suivant l'une des revendications 1 à 4, dans lequel un équipement (24) d'entraînement est disposé sur le support (1) de base et est relié au chariot (20) longitudinal.

6. Appareil modulaire de diagnostic par rayons X suivant la revendication 5, dans lequel un moteur (24A) de l'équipement (24) d'entraînement est disposé à l'intérieur du support (1) de base.

7. Appareil modulaire de diagnostic par rayons X suivant l'une des revendications 1 à 6, dans lequel les moyens de couplage de composant comprennent un palier (14) fixe supplémentaire et un palier (15) libre supplémentaire disposés sur le support (1) de base.

8. Appareil modulaire de diagnostic par rayons X suivant l'une des revendications 1 à 7, dans lequel un équipement (19) d'entraînement supplémentaire, pour le composant, est disposé sur le support (1) de base.

9. Appareil modulaire de diagnostic par rayons X suivant la revendication 8, dans lequel des composants différents peuvent être réglés au moyen de l'équipement (19) d'entraînement supplémentaire.

10. Appareil modulaire de diagnostic par rayons X suivant l'une des revendications 7 à 9, dans lequel l'équipement (19) d'entraînement supplémentaire est disposé entre le palier (14) fixe et le palier (15) libre.

11. Appareil modulaire de diagnostic par rayons X suivant la revendication 9 ou 10, dans lequel le dispositif (33) de soutien pour l'objet à examiner est monté sur un autre palier (32) fixe.

12. Appareil modulaire de diagnostic par rayons X suivant la revendication 11, dans lequel le dispositif (33) de soutien peut être réglé sur l'autre palier (32) fixe.

13. Appareil modulaire de diagnostic par rayons X suivant l'une des revendications 1 à 12, dans lequel le dispositif (33) de soutien est disposé au-dessus du récepteur (28) de rayons.

14. Appareil modulaire de diagnostic par rayons X suivant l'une des revendications 1 à 13, dans lequel le récepteur de rayons (13) et/ou le dispositif (33) de soutien engagent un palier (15) libre qui est disposé sur un montant (16) longitudinal orienté au moins approximativement parallèlement à l'axe longitudinal du support (1) de base et distant du support (1) de base.

15. Appareil modulaire de diagnostic par rayons X suivant la revendication 14, dans lequel le montant (16) longitudinal est relié au support (1) de base par l'intermédiaire d'un montant transversal.

16. Appareil modulaire de diagnostic par rayons X suivant la revendication 15, dans lequel le montant transversal engage les moyens (31) de couplage de soutien formés sur le côté frontal du support (1) de base.

17. Appareil modulaire de diagnostic par rayons X suivant la revendication 15 ou 16, dans lequel un autre montant transversal engage les moyens (32) de couplage de soutien formés sur l'autre côté frontal du support de base et est relié au montant (16) longitudinal.

18. Appareil modulaire de diagnostic par rayons X suivant l'une des revendications 1 à 17,
dans lequel un récepteur (28) de rayons est disposé au-dessus du chariot (20) longitudinal par l'intermédiaire d'une tour (27) de compression disposée sur le chariot (20) longitudinal,
et dans lequel une source (26) de rayons est prévue en dessous du chariot (20) longitudinal, en étant disposée sur le chariot (20) longitudinal à distance du support (1) de base.

19. Appareil modulaire de diagnostic par rayons X suivant l'une des revendications 1 à 17, dans lequel une source (26) de rayons est disposée au-dessus du chariot (20) longitudinal par l'intermédiaire d'une colonne (21) en forme de potence disposée sur le chariot (20) longitudinal.

20. Appareil modulaire de diagnostic par rayons X suivant l'une des revendications 1 à 19, dans lequel un organe (29) porteur pour un arceau (30) de radiographie en forme de C est monté sur le chariot (20) longitudinal, organe au moyen duquel l'arceau (30) de radiographie en forme de C peut être réglé au moins le long de son pourtour.

21. Appareil modulaire de diagnostic par rayons X suivant la revendication 20, dans lequel l'arceau (30) en forme de C de radiographie peut, au moyen de l'organe (29) porteur, être réglé autour d'un axe horizontal et/ou d'un axe vertical.

22. Appareil modulaire de diagnostic par rayons X suivant l'une des revendications 1 à 21, dans lequel le support est réalisé sous forme de statif plafonnier.

23. Appareil modulaire de diagnostic par rayons X suivant l'une des revendications 1 à 21, dans lequel le support est réalisé sous forme de socle (5).

24. Appareil modulaire de diagnostic par rayons X suivant la revendication 23, dans lequel on peut produire au moyen du socle (5) un réglage en hauteur du support (1) de base.

25. Appareil modulaire de diagnostic par rayons X suivant la revendication 23 ou 24, dans lequel on peut produire au moyen du socle (5) un pivotement du support (1) de base autour d'un axe horizontal.

26. Appareil modulaire de diagnostic par rayons X suivant l'une des revendications 23 à 25, dans lequel le socle (5) engage les moyens (6) de couplage de support prévus sur le dessous et/ou sur au moins une des parois latérales du support (1) de base.

27. Appareil modulaire de diagnostic par rayons X suivant l'une des revendications 23 à 26, dans lequel le socle (5) est disposé au-dessous du support (1) de base.

28. Appareil modulaire de diagnostic par rayons X suivant l'une des revendications 23 à 26, dans lequel le socle (5) est disposé sur le côté du support (1) de base et engage les moyens (2) de couplage de support par l'intermédiaire d'un élément (3) de liaison.

29. Procédé de fabrication de séries différentes d'appareils de diagnostic par rayons X,
suivant lequel on utilise pour toutes les séries un support (1) de base modulaire de construction identique et un dispositif (33) de soutien modulaire de construction identique,
suivant lequel chaque support (1) de base est, en tant que module central, préparé en différents points de couplage pour l'accouplement de tous les modules apparaissant dans les séries à réaliser,
et suivant lequel le dispositif (33) de soutien et, en fonction de la série respective, des modules supplémentaires sont installés sur le support (1) de base,
les modules supplémentaires comprenant un support pour le support (1) de base et/ou un composant d'un équipement enregistreur de radiographie et/ou un chariot (20) longitudinal.

30. Procédé de fabrication suivant la revendication 29, suivant lequel les séries sont des séries de types d'appareils différents les uns des autres, notamment une série d'appareils de diagnostic par rayons X sur table, une série d'appareils de diagnostic par rayons X sous table et une série d'appareils de diagnostic par rayons X à arceau en forme de C.

31. Procédé de fabrication suivant la revendication 29 ou 30, suivant lequel les séries sont des séries du même type d'appareils avec un équipement plus ou moins complet ou confortable.
